# EUROPÄISCHE PATENTANMELDUNG

(11) **EP 2 698 121 A1**
(43) Veröffentlichungstag der Anmeldung: **19.02.2014**
(21) Anmeldenummer: 13004016.5
(22) Anmeldetag: 10.08.2013
(51) Int. Cl.: A61B 17/70

(54) **Polyaxialer Konnektor für Wirbelsäulen-Fixationssystem und Wirbelsäulen-Fixationssystem**

(30) Priorität: 16.08.2012 DE 102012016294
(71) Anmelder: spontech spine intelligence group AG, 70182 Stuttgart (DE)
(72) Erfinder: Taddia, Lino, D-78333 Stockach (DE); Pabst, Martin, D-70191 Stuttgart (DE)
(74) Vertreter: Schwanhäußer, Gernot

(57) **Zusammenfassung**

Der erfindungsgemäße polyaxiale Konnektor (2) besteht im Wesentlichen aus einem querverbindenden Konnektorelement (9) mit zwei an seinen jeweiligen Enden befindlichen polyaxialen Verbindungsanordnungen (7, 8) zur Aufnahme eines Pedikelschraubenkopfes (18) einerseits und eines Verbindungsstabes (3) andererseits. Mit Hilfe eines in der Aufnahme für den Verbindungsstab (3) vorgesehenen einschraubbaren Feststellelementes (25) und eines im Konnektorelement (9) zwischen den Verbindungsanordnungen (7, 8) dreh- und längsbeweglich gelagerten Klemmstückes (28) kann eine vollständige kraftschlüssige Verbindung zwischen Verbindungsstab (3) und Pedikelschraube (1) erzielt werden. Ein Lösen des Feststellelementes (25) hebt die Fixierung auf.

Der polyaxiale Konnektor ermöglicht trotz hoher Flexibilität mit acht Freiheitsgraden der Bewegung im nicht fixierten Zustand ein einfaches und schnelles Festlegen in nur einem Schritt, er besteht aus verhältnismäßig wenig Bauteilen und es werden im nicht fixierten Zustand weder Kräfte noch Momente zwischen der Pedikelschraube und dem Verbindungsstab aufgebaut.

## Beschreibung

Die vorliegende Erfindung betrifft einen polyaxialen Konnektor für Wirbelsäulen-Fixationssysteme. Wirbelsäulen-Fixationssysteme werden im Bereich der Wirbelsäulenchirurgie angewandt und dienen zur Stabilisierung der Wirbelsäule, insbesondere bei Traumata und degenerativen Erkrankungen, die eine Korrektur, Stabilisierung oder Fixierung der Wirbelsäule erfordern. Aus dem Stand der Technik sind bereits zahlreiche Wirbelsäulen-Fixationssysteme bekannt, wobei diese bekannten mechanischen Systeme üblicherweise Verankerungselemente in Form von Pedikelschrauben und Verbindungsstäben, die die Köpfe der Pedikelschrauben miteinander verbinden, umfassen. Bekannte Pedikelschrauben weisen jeweils einen zur Knochenverankerung vorgesehenen Schaft und einen mit einem Verbindungsstab verbindbaren, tulpenartigen Aufnahmekopf auf. Diese "Tulpen" besitzen einen im Wesentlichen U-förmigen Querschnitt, wobei sie an ihrem Grund fest oder durch eine kugelgelenkartige Anordnung ("polyaxiale Tulpen") unmittelbar mit dem Schaft der Pedikelschraube verbunden sind, und zwei, einen Kanal zur Aufnahme jeweils eines Verbindungsstabes bildende freie Schenkel mit einem Innengewinde, um ein Schrauben eines Festellelementes gegen den Verbindungsstab zu ermöglichen. Auf diese Weise kann der Verbindungsstab mit einer Pedikelschraube durch direktes Festklemmen verbunden werden. Übliche Wirbelsäulen-Fixationssysteme benutzen dabei starre, durch die Pedikelschrauben an der Wirbelsäule befestigte Verbindungsstäbe. Da die Wirbelsäule jedes Patienten einen anderen anatomischen Aufbau, inklusive unterschiedlicher Knochenformen und Knochendichten, aufweist, werden der genaue Ort der Pedikelschrauben und anderer Teile eines Wirbelsäulen-Fixationssystems, einschließlich der korrekten Form der Verbindungsstäbe, erst während der Operation festgelegt, d.h., während der Patient bereits auf dem Operationstisch liegt. Das Nachformen von Verbindungsstangen, so dass diese an den dreidimensionalen Aufbau der Wirbelsäule eines jeden Patienten angepasst sind, ist aber sehr schwierig, zeitaufwendig und kann zu Fehlern führen. Sogar wenn die dreidimensionale anatomische Ausrichtung von Pedikelschrauben bereits vor der Operation bestimmt wird, ist es oft notwendig, diese in den Pedikeln neu zu orientieren und/oder ihre Eindringtiefe nochmals zu justieren, da die zum Festklemmen des Verbindungsstabes ausgeübte Kraft bei den oben geschilderten Systemen direkt auf die Pedikelschraube einwirkt. Dies erfordert nicht selten eine komplette Entfernung einer Pedikelschraube, insbesondere wenn die Wirbelsäulen-Deformation den Erwartungen nicht entspricht oder der erforderliche Raum für die Pedikelschraube nicht ausreicht. Das Entfernen, Auswechseln oder Nachziehen von Pedikelschrauben gefährdet aber die leicht zerbrechliche Knochenstruktur der Pedikel um den Schraubenschaft herum und benötigt zusätzliche Operationszeit.

Um die oben genannten Nachteile zu beseitigen wurden bereits Wirbelsäulen-Fixationssysteme entwickelt, die zwischen der Aufnahme für den Verbindungsstab und der Pedikelschraube einen querverbindenden Konnektor, derart vorsehen, dass der Verbindungsstab in seitlichem Versatz zur longitudinalen Ausrichtung der Pedikelschraube befestigt werden kann. Die Verbindung zwischen der Pedikelschraube und dem Verbindungsstab kann dabei nach dem Einsetzen der Pedikelschraube erfolgen, sodass kein zusätzliches Nachdrehen oder neues Ausrichten der Pedikelschraube erforderlich ist. Hierdurch eventuell verursachte Lockerungen werden mithin vermieden.

### Stand der Technik

Aus der US 2003/0045879 A1 ist ein derartiger querverbindender Konnektor für Wirbelsäulen-Fixationssysteme bekannt, der sowohl eine Rotation um die longitudinale Achse der Pedikelschraube bei weitgehender Kippmöglichkeit aus der Rotationsebene durch eine polyaxiale Verbindung erlaubt als auch eine Einstellung des seitlichen Abstands zwischen der Pedikelschraube und dem Verbindungsstab mit gleichzeitiger Drehmöglichkeit um die Längsachse eines vom Pedikelschraubenkopf weg zeigenden, stabförmigen Fortsatzes des Konnektors selbst. Dabei ist eine tulpenförmige Aufnahme für den Verbindungsstab auf dem stabförmigen Fortsatz des Konnektors verschieblich und drehbar gelagert und durch das in der tulpenförmigen Aufnahme befindliche Feststellelement für den Verbindungsstab festlegbar. Die Rotations- und Kippbewegung wird durch einen kugelgelenkartigen zweiten Teil des Konnektors erreicht, wobei an dessen Unterseite ein Lager angeordnet ist, das einen kugelsymmetrischen Pedikelschraubenkopf aufnehmen kann, und an dessen Oberseite ein zweites Feststell- bzw. Klemmelement zur Fixierung existiert.

Da der Verbindungsstab vor seiner Fixierung in der Aufnahme gedreht und verschoben werden kann ergeben sich für diesen Konnektor mithin 7 Freiheitsgrade der Bewegung im unfixierten Zustand.

Die US 2007/0288004 A1 offenbart einen querverbindenden Konnektor für Wirbelsäulen-Fixationssysteme mit einem Kopfteil, der auf einen kugelsymmetrischen Schraubenkopf einer Pedikelschraube aufsetzbar ist, so dass eine kugelgelengartige Verbindung mit drei Freiheitsgraden der Rotation gegeben ist. Diese kann durch ein Festellelement polyaxial festgelegt werden. Der Kopfteil ist mit einem Arm verbunden auf dem eine tulpenartige Aufnahme für den Verbindungsstab drehbar gelagert ist und zwar derart, dass eine zusätzliche Rotation in der Ebene möglich ist, in der der Arm liegt. Zur Fixierung des Verbindungsstabes innerhalb der tulpenartigen Aufnahme wird ein zweites Feststellelement benötigt.

Mit den Möglichkeiten des Drehens und des Verschiebens der Verbindungsstange in der Aufnahme ergeben sich damit 6 Freiheitsgrade der Bewegung im nicht fixierten Zustand.

Ein weiterer, aus der US 2007/0238335 A1 bekannter querverbindender Konnektor besitzt im Gegensatz zu den bisher aufgezeigten Konnektoren keine direkte polyaxiale Verbindung mit der Pedikelschraube. Die Pedikelschraube weist keinen kugelförmigen Kopf sondern stattdessen ein Gewinde auf, um ein mit einem Befestigungsloch versehenes Anschlussteil des Konnektors nach dessen Aufstecken mittels einer Befestigungsmutter festschrauben zu können. Vor dem Befestigen ergibt sich hieraus eine Rotationsmöglichkeit des Verbinders um die longitudinale Achse der Pedikelschraube, wobei die Befestigungshöhe des Anschlussteils durch einen am Schraubenschaft befindlichen ringförmigen Absatz, dessen Außendurchmesser größer als der Innendurchmesser des Befestigungslochs ist, fest vorgegeben ist. Das derart befestigte Anschlussteil des Konnektors ist über eine drehbare Kupplung fest mit einer Klemmvorrichtung für den Verbindungsstab verbunden, wodurch eine Drehung um eine zur longitalen Ausrichtung der Pedikelschraube liegende Querachse, die durch die Längsachse des querverbindenden Konnektors verläuft, ermöglicht wird. Die Klemmvorrichtung beinhaltet ein Feststellelement für den Verbindungsstab, das mittels eines in der Klemmvorrichtung verschieblich angeordneten Haltebügels und ineinandergreifender Verzahnungen eines Kupplungsteils zwischen dem Anschlussteil und der Klemmvorrichtung gleichzeitig zur Blockierung der Rotationsbewegung um die Längsachse des querverbindenden Konnektors dient.

In Anbetracht dessen, dass der Verbindungsstab vor einer Fixierung in der Klemmvorrichtung verschoben und gedreht werden kann, ermöglicht dieser Konnektor daher 4 Freiheitgrade der Bewegung im nicht fixierten Zustand auf.

Die vorgenannten querverbindenden Konnektoren besitzen 4, 6 oder 7 Freiheitsgrade der Bewegung im nicht fixierten Zustand und benötigen zur endgültigen Festlegung jeweils zwei Feststellelemente. Jedes durch einen Chirurgen während einer Operation einzustellende Feststellelement kompliziert aber nicht nur ein Fixationssystem als solches, sondern verlängert auch zusätzlich die Einstell- bzw. Operationszeit.

Um hier Abhilfe zu schaffen wurden bereits Wirbelsäulen-Fixationssysteme mit querverbindenden Konnektoren entwickelt, die trotz mehrerer Freiheitsgrade der Bewegung im nicht fixierten Zustand mit nur einem Feststellelement auskommen, um in den fixierten Zustand überführt zu werden.

Ein solcher querverbindender Konnektor mit 5 Freiheitsgraden der Bewegung im nicht fixierten Zustand und nur einem Feststellelement ist aus der US 2007/0156142 A1 bekannt.

Dieser Konnektor ähnelt in seiner Ausführung dem letztgenannten Querverbinder mit vier Freiheitsgraden der Bewegung im nicht fixierten Zustand mit dem wesentlichen Unterschied, dass die Verbindung zwischen der Pedikelschraube und dem Konnektor durch eine andersartige Klemmvorrichtung ausgeführt ist, die einen zylindrischen Pedikelchraubenkopf ohne Gewinde ringartig umschließt und gleit- und befestigbar daran anbringbar ist, so dass neben der Rotationsbewegung um die longitudinale Achse der Pedikelschraube zusätzlich auch eine Höhenverstellbarkeit in deren Richtung gegeben ist. Die Festlegung dieser Klemmvorrichtung an der Pedikelschraube erfolgt über ein daran angeformtes in Längsrichtung zusätzlich verschiebliches Kupplungselement gleichzeitig mit dem Anziehen des Feststellelementes für den Verbindungsstab in der anderen Klemmvorrichtung, sodass eine Überführung in den fixierten Zustand mit nur einem Feststellung möglich ist, also in einem Schritt erfolgen kann.

Weiterer derartige querverbindende Konnektoren können der DE 10 2004 056 091 B4 entnommen werden. Diese bestehen im Prinzip aus zwei Klemmvorrichtungen, die über ein kardangelenkartiges und zusätzlich längsverschiebliches Kupplungsstück miteinander in Verbindung stehen. Während die erste Klemmvorrichtung zur Verbindung mit einem Verbindungsstab eines Wirbelsäulen-Fixationssystems dient, dient die zweite Klemmvorrichtung zur Verbindung mit einem Pedikelschraubenkopf. Wenn die zweite Verbindungsvorrichtung wie vorgeschlagen zur Aufnahme und Verbindung mit einem kugelkopfartigen Pedikelschraubenkopf ausgebildet ist, ergibt sich eine maximale Anzahl von 8 Freiheitsgraden der Bewegung im unfixierten Zustand, wenn man die Dreh- und Verschiebemöglichkeiten des Verbindungsstabes in der ersten Klemmvorrichtung vor dem Fixieren einschließt. Um alle freien Bewegungsmöglichkeiten in einem Schritt zu fixieren, genügt das Anziehen eines Spanngliedes im Kupplungsstück.

Der hier aufgezeigte querverbindende Konnektor ist zwar auf Grund seiner 8 Freiheitsgrade der Bewegung äußerst flexibel und erfüllt die Bedingung der Einstellbarkeit in einem Schritt, er ist indes auf Grund der Vielzahl seiner Einzelteile recht aufwendig gestaltet und durch seine mit Rillen versehenen Kupplungsteile schmutzanfällig und schwer zu reinigen.

Eine Verbesserung in diesem Sinne stellt der aus der WO 2010/108655 A2 bekannte querverbindende Konnektor dar.

Dieser besteht im Wesentlichen aus einem länglichen, sog. Kopfelement, das um einen zylindrischen Pedikelschraubenkopf drehbar und höhenverstellbar gelagert ist, so dass sich das Kopfelement in seiner länglichen Ausdehnung in verschiedenen, rechtwinklig zur longitudinalen Achse der Pedikelschraube erstreckenden Ebenen bewegen kann, und aus einer polyaxial beweglichen Tulpe als Aufnahme- und Verbindungsteil für einen Verbindungsstab. Die polyaxiale Tulpe ist an einem im Abstand von der Pedikelschraube befindlichen Teil des Kopfelementes angebracht und zeigt mit ihrem u-förmigen Aufnahmeteil vom Schaft der Pedikelschraube weg. Über ein in der polyaxialen Tulpe befindliches Klemmelement in Form einer Spannschraube kann nicht nur der Verbindungsstab sondern auch die Tulpe selbst und die dreh- und höhenverstellbare Verbindung des Konnektors mit dem Pedikelschraubenkopf gleichzeitig fixiert werden. Dies erfolgt über rechtwinklig zueinander stehende Bolzen mit schrägen Auflageflächen und einer Einwegkupplung, die in Bohrungen im Inneren des Kopfelementes untergebracht sind. Diese bekannte Konstruktion eines querverbindenden Konnektors eröffnet nicht nur durch seine zahlreichen polyaxialen Rotationsmöglichkeiten und der möglichen Höhenverstellbarkeit 7 Freiheitsgrade der Bewegung im nicht fixierten Zustand sondern erlaubt auch ein Befestigen eines Verbindungsstabes, der direkt oder teilweise über dem Pedikelschraubenkopf selbst verläuft.

Nachteilig an diesem bekannten, bereits äußerst flexiblen Aufbau ist aber, dass zur Lösung der Einwegkupplung ein spezielles, in das Kopfelement einzuführendes Werkzeug benötigt wird.

Die US 80 66 746 B2 zeigt und beschreibt einen Konnektor, bei welchen eine Schraube auf ein Verbindungselement wirkt, welches seinerseits auf ein Zwischenteil mit Innengewinde wirkt, wobei in den Innengewinde ein weiteres Zwischenteil eingeschraubt ist, welches auf eine Pedikelschraube wirkt. Das Eindrehen der Schraube überträgt die Kräfte auf die Bauteile in der angegebenen Reihenfolge und fixiert Verbindungselement und Pedikelschraube, wobei die Anzahl der Freiheitsgrade der möglichen Stellungen 3 ist.

### Zugrundeliegendes Problem und Lösung

Diesen Mangel zu beseitigen, gleichzeitig ein Maximum an Flexibilität beizubehalten und weiterhin eine Fixierung in einem Schritt zu ermöglichen liegt der vorliegenden Erfindung als Aufgabe zugrunde.

Diese Aufgabe wird durch einen polyaxialen Konnektor nach dem vorliegenden Patentanspruch 1 gelöst.

### Erreichte Vorteile

Die mit der Erfindung erreichten Vorteile liegen insbesondere in der hohen Flexibilität trotz verhältnismäßig geringer Bauteilanzahl, wobei im nicht fixierten Zustand 8 Freiheitsgrade der Bewegung zur Verfügung stehen, in einer einfachen und schnellen Überführung von einem beweglichen in einen vollständig fixierten Zustand und vice versa und der vollständigen Aufnahme des notwendigen Verklemm- und Entsperrmechanismus im Inneren des Konnektorelementes. Durch die hohe Anzahl an geometrischen Freiheiten, insbesondere durch die Erhöhung der polyaxialen Verbindungsmöglichkeiten ist es möglich, einen Verbindungsstab in einer innerhalb eines limitierten Abstands beliebigen Lage gegenüber dem Wirbelkörper auszurichten und anschließend mit gutem Kraftschluss zu fixieren. Die Fixierung der Lage der Wirbelkörper zueinander kann weitgehend unabhängig von der Geometrie des Verbindungsstabes gewählt werden. Es ist deshalb möglich, die Wirbelkörper in der vom Chirurgen gewünschten Lage zu fixieren, ohne dass diese Lage durch die geometrische Gestalt der posterioren Stäbe beeinflusst wird. Der Konnektor ist so ausgelegt, dass im nicht fixierten Zustand und während des Vorgangs der Fixierung keine Kräfte oder Momente zwischen Pedikelschraube und Verbindungsstab innerhalb dieses Systems aufgebaut werden. Durch die gegebene Konstruktion verringert sich die initiale Einleitung von Kräften auf die Schnittstelle Pedikelschraube/Pedikel substanziell. Diese Verringerung der Belastung führt dazu, dass Folgeeffekte wie Lockerungen von Pedikelschrauben und Schraubenbrüche weniger häufig auftreten.

Weitere vorteilhafte Aus- oder Weiterbildungen des polyaxialen Konnektors finden sich in den Unteransprüchen 2 bis 8, Verwendungen der beanspruchten polyaxialen Konnektoren in den mit den Patentansprüchen 9 und 10 jeweils beanspruchten Wirbelsäulen-Fixationssystemen.

### Ausführungsbeispiel

Ausführungsbeispiele der Erfindung, die weitere Vorteile und Besonderheiten erkennen lassen, werden in den Zeichnungen dargestellt und im Folgenden näher beschrieben. Zum besseren Verständnis wird zunächst von einem erfindungsgemäßen Wirbelsäulenfixationssystem(5) ausgegangen, bei dem die erfindungsgemäßen Konnektoren(2) Verwendung finden.

Es zeigen:
- Figur 1:: eine perspektivische Ansicht auf ein erfindungsgemäßes, in einem Abschnitt der menschlichen Wirbelsäule implantiertes Wirbelsäulen-Fixationssystem (5)
- Figur 2:: das in Figur 1 dargestellte erfindungsgemäße Wirbelsäulen-Fixationssystem (5) in Draufsicht
- Figur 3:: das in Figur 1 dargestellte erfindungsgemäße Wirbelsäulen-Fixationssystem (5) in seitlicher Ansicht
- Figur 4:: einen erfindungsgemäßen Konnektor (2) mit eingerasteter Pedikelschraube (1) im Schnitt
- Figur 5:: einen erfindungsgemäßen Konnektor (2) mit eingerasteter Pedikelschraube (1) in Ansicht
- Figur 6:: ein Klemmstück (28) in perspektivischer Ansicht
- Figur 7:: einen erfindungsgemäßen Konnektor (2) im horizontalen Teilschnitt
- Figuren 8, 9:: die Freiheitsgrade der Bewegung des polyaxialen Konnektors (2) im nicht fixierten Zustand

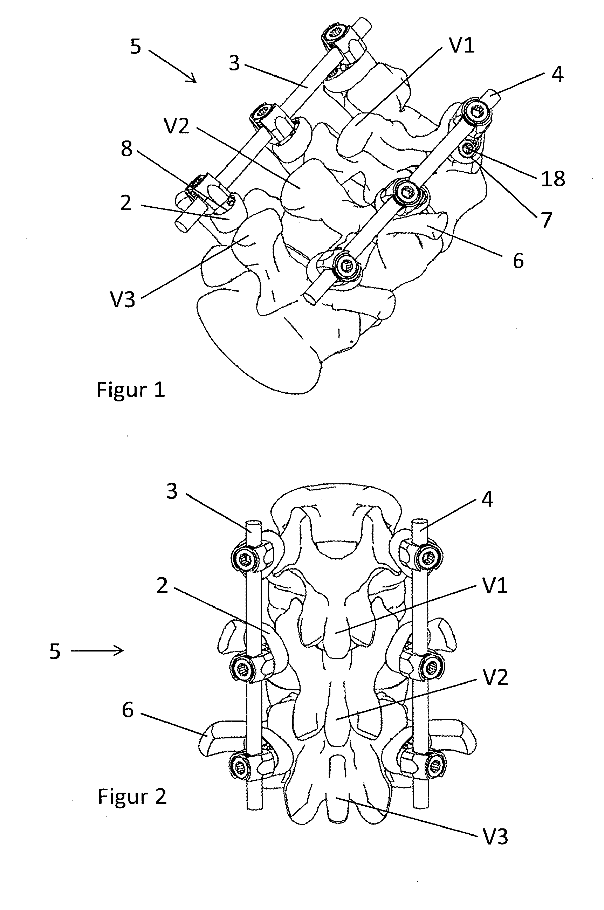

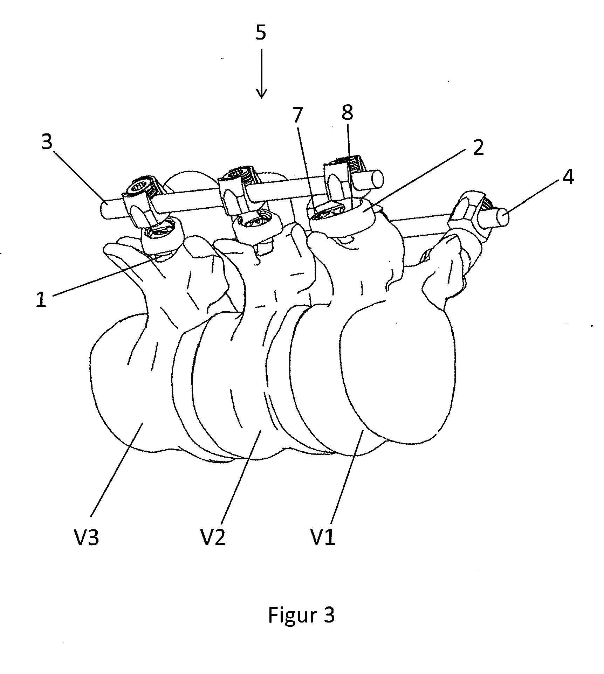

In den Figuren 1 bis 3 ist jeweils beispielhaft ein in einem Abschnitt der menschlichen Wirbelsäule implantiertes, erfindungsgemäßes Wirbelsäulen-Fixationssystem (5) dargestellt, in Figur 1 in perspektivischer Ansicht, in Figur 2 in Draufsicht und in Figur 3 in seitlicher Ansicht. Der Abschnitt umfasst jeweils drei aufeinanderfolgende Wirbelknochen (V1, V2, V3), wobei der Einfachheit halber die angrenzenden Wirbelknochen in den Figuren 1 bis 3 nicht eingezeichnet sind.

Das Wirbelsäulen-Fixationssystem(5) umfasst zwei Verbindungsstäbe (3,4), welche sich beidseitig der Wirbelsäule in longitudinaler Richtung der Wirbelsäule erstrecken. Im aufgezeigten Beispiel sind die Verbindungsstäbe (3,4) als gerade, biegesteife Stangen dargestellt, die aus einem geeigneten Werkstoff, z.B. Titan, bestehen können. Bei anderen Ausführungen können diese Verbindungsstäbe (3,4) gekrümmt oder anderweitig vorgebogen sein. Die Verbindungsstäbe (3,4) können einen runden, ovalen oder polygonalen Querschnitt aufweisen.

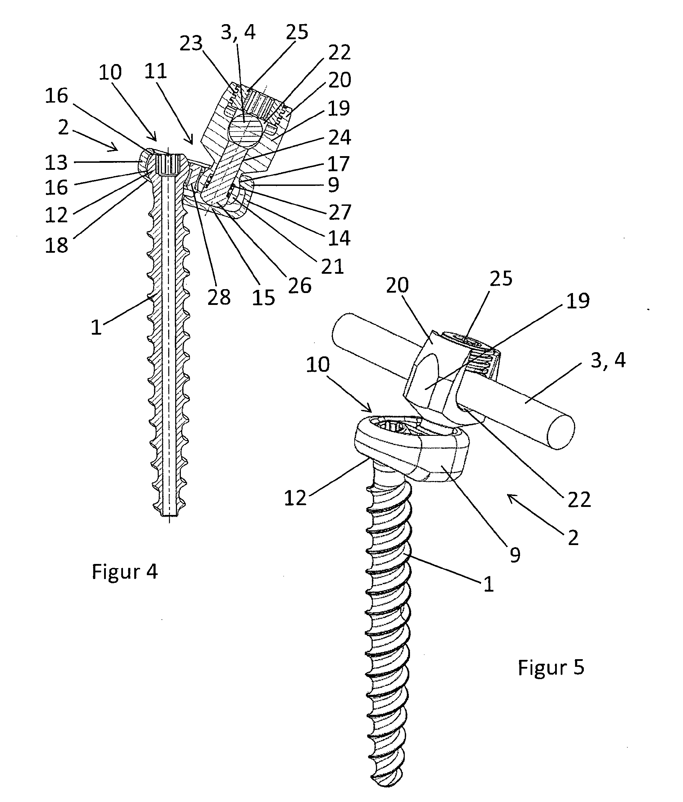

Die Verbindungsstäbe (3,4) sind an den Wirbelknochen (V1, V2, V3) mit Hilfe von Pedikelschrauben(1), welche in den jeweiligen Pedikeln (6) der Wirbelknochen (V1, V2, V3) eingeschraubt sind, und polyaxialen Konnektoren (2), die die Pedikelschrauben (1) mit den Verbindungsstäben (3,4) verbinden, befestigt. Wie aus der nachfolgenden Beschreibung der erfindungsgemäßen polyaxialen Konnektoren (2) und unter Bezug auf die Figuren 4 bis 9 ersichtlich, besitzen diese polyaxialen Konnektoren (2) jeweils zwei Schnittstellen in Form von polyaxialen Verbindungsanordnungen (7,8). Die hieraus sich ergebende Anzahl von acht Freiheitsgraden der Bewegung ermöglicht vielfältige Ausrichtungen zwischen einem Verbindungsstab (3,4) und einer Pedikelschraube (1). So können beispielsweise die geraden, biegesteifen Verbindungsstäbe (2,3) mit den Pedikelschrauben (1)verbunden werden, obwohl letztere weder parallel zueinander noch in einer Linie ausgerichtet sind, wie es am besten aus Figur 2 ersichtlich ist. Weiterhin können die Verbindungsstäbe(3,4) direkt über den Pedikelschraubenköpfen (18) oder querab davon verlaufen.

Figur 4 zeigt im Schnitt den erfindungsgemäßen, bereits mit einer Pedikelschraube (1) verbundenen polyaxialen Konnektor (2), bestehend aus einem Konnektorelement (9), einer Tulpe (19) mit Tulpenkopf (20) und einem Kugelkopf (21). Im Tulpenkopf(20) ist ein Schlitz (22) zur Aufnahme eines Verbindungsstabes (3,4) vorhanden. Im Tulpenkopf (20) ist weiterhin ein Gewinde (23) zum Einschrauben eines Feststellelementes (25) mit Aussengewinde ausgebildet. Am unteren Ende geht der Tulpenkopf (20) in den Kugelkopf (21) über. Dieser besitzt eine zentrale, abgesetzte Bohrung (24) zur axialen Führung eines federbelasteten Druckstößels (26). Die Feder (27) wirkt als Druckfeder, welche sich einerseits in der abgesetzten Bohrung (24) und andererseits am Kragen des Druckstößels (26)abstützt.

Wie aus Figur 5 ersichtlich sind alle äußeren Ecken und Kanten des Konnektorelementes (9) abgerundet, um die Handhabung zu verbessern und die Verletzungsgefahr zu verringern. Auch ist in dieser Ansicht zu erkennen, dass das Konnektorelement (9) einen im Bereich der ersten Aufnahme (10) etwa von der Mitte zur Seite hin schräg nach oben verlaufenden Abschnitt (12) des Gehäusebodens (15) aufweist, um einen größeren Schwenkwinkel der Pedikelschraube (1) bzw. ein leichteres Einführen des Pedikelschraubenkopfes (18) zu ermöglichen.

Wie weiterhin Figur 4 zeigt, ist die linke Gehäusewand (13) des Konnektorelements (9) U-förmig zur Anlage des Pedikelschraubenkopfes (18) ausgebildet. Die entgegengesetzte Gehäusewand (14) des Konnektorelements (9) ist im Wesentlichen ebenfalls U-förmig ausgeführt und geht in den Gehäuseboden (15) über. Am oberen Rand besitzt sie einen innen liegenden Wulst oder eine halbringförmige Verdickung als Klemmfläche (17).

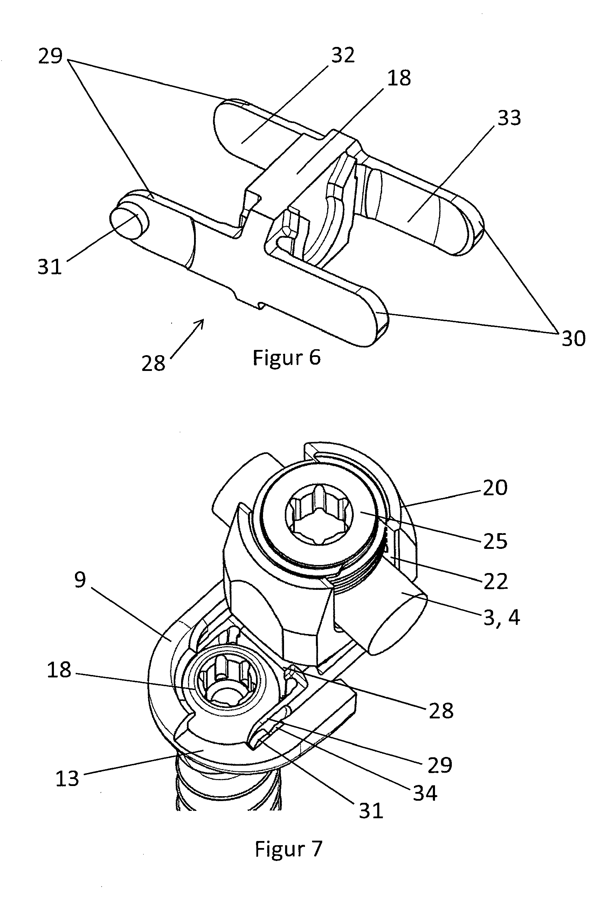

Zwischen Kugelkopf (21) und Pedikelschraubenkopf (18)befindet sich ein Klemmstück (28), welches in Draufsicht eine H-förmige Grundstruktur mit Laschen (29) und (30), wie in Figur 6 dargestellt, aufweist. Die parallelen Laschen (29) sind an ihren gegenüberliegenden, inneren Flächen (32) zur Anlage an den Pedikelschraubenkopf (18) zylinderförmig geformt, damit der Pedikelschraubenkopf (18) linienförmig anliegt, wenn er zwischen den Laschen (29) einrastet. Zur Erleichterung des Einrastens sind die Laschen (29) federelastisch. Die Außenflächen der Laschen (29) besitzen diametral auf einer geometrischen Achse einstückig ausgebildete, zylindrische Zapfen(31).

Die den Laschen (29) gegenüberliegenden Laschen (30) besitzen auf den inneren Flächen (33) ebenfalls eine zylinderförmige Gestalt, die mit dem Kugelkopf (21) in Linienberührung bringbar ist. Auch der Kugelkopf (21) rastet ein, wobei die Laschen (29) dazu beitragen, das Klemmstück (28) in der richtigen Position zu halten solange der Pedikelschraubenkopf (18) nicht eingerastet ist, wozu diese Laschen (30) ebenfalls federelastische Eigenschaften aufweisen. Die zylinderförmigen Flächen (33) gehen am äußeren Ende der jeweiligen Laschen(30)in eine Kugelform über, angepasst an den Kugelkopf (21).

Figur 7 zeigt eine horizontale Führungsnut (34) im Konnektorelement (9). Diese Führungsnut (34) nimmt die Zapfen (31) des Klemmstückes (28) mit Spiel auf und ermöglicht eine horizontale, lineare Bewegung des geführten Teils des Klemmstückes (28).

Im nicht fixierten bzw. unverspannten Zustand ist die Pedikelschraube (1) über ihren Pedikelschraubenkopf (18) zwischen den Laschen (29) des Klemmstückes (28) eingerastet und befindet sich in der ersten Aufnahme (10) des Konnektorelementes (9). In diesem Zustand ist die Pedikelschraube (1) frei schwenkbar und drehbar. Der Kugelkopf (21) der Tulpe (19) ist zwischen den Laschen (30) des Klemmstückes (28) eingerastet und lässt Verschwenken und Verdrehen zu. In den Schlitz (22) der Tulpe (19) ist ein Verbindungsstab (3,4) eingelegt und das Feststellelement (25) soweit eingeschraubt, dass der Verbindungsstab (3,4) nicht mehr in Längsrichtung des Schlitzes (22) aus dem Schlitz (22) herausgenommen werden kann. Dem Chirurg ist es nun ermöglicht, alle Teile eines Wirbelsäulen-Fixationssystemes (5) in die gewünschte Position zu bringen, die er für die zu operierende Wirbelsäule benötigt. Ist diese Position erlangt, reicht ausschließlich das Einschrauben der Feststellelemente (25) aller einzelner Konnektoren (2) aus, um das gesamte Fixationssystem (5) kraftschlüssig zu befestigen.

Durch das Eindrehen des Feststellelementes (25) drückt dieses auf den Verbindungsstab (3,4). Dieser verschiebt seinerseits den Druckstößel (26) gegen den Gehäuseboden (15). Die Reaktionskraft aufgrund der Einschraubkraft bringt den Kugelkopf (21) der Tulpe (19) zur Anlage an der rechten U-förmigen Gehäusewand(14) des Konnektorelementes (9), wodurch diese Teile fixiert sind. Da die Anlage des Kugelkopfes (21) durch den innenliegenden Wulst am oberen Rand der Gehäusewand (14) bzw. die Klemmfläche (17) auch eine horizontale Kraftkomponente in Richtung des Pedikelschraubenkopfes (18) bewirkt, erfolgt eine geringe Verschiebung des Kugelkopfes (21) horizontal in Richtung der Pedikelschraube (1), wobei zuerst der Kugelkopf (21) in kraftschlüssige Anlage an das Klemmstück(28) gelangt. Dieses wiederum kommt in kraftschlüssige Anlage an den Pedikelschraubenkopf (18), welcher seinerseits zur Anlage an einer Klemmfläche (16) an der U-förmigen Gehäusewand(13) des Konnektorelementes (9) kommt. Somit bewirkt ausschließlich das Einschrauben des Feststellelementes (25) eine vollständige, kraftschlüssige Verbindung zwischen einem Verbindungsstab (3,4)und einer Pedikelschraube (1). Ein Lösen des Feststellelementes (25) hebt die gesamte Fixierung auf.

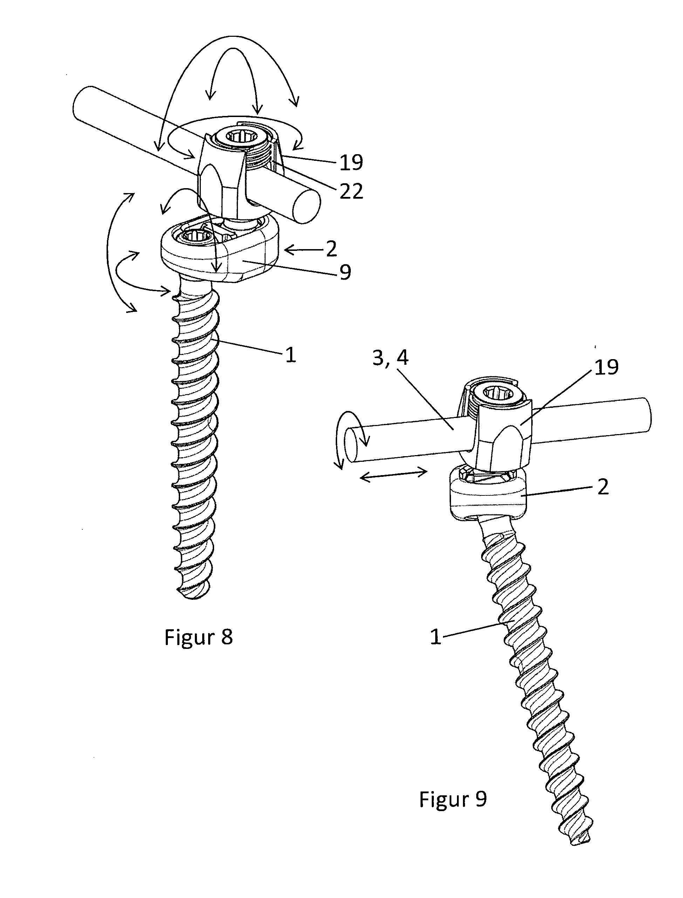

In den Figuren 8 und 9 sind die möglichen Freiheitsgrade der Bewegung des erfindungsgemäßen Konnektors (2)dargestellt. So kann die Tulpe (19) im Konnektorelement (9) um 360° um ihre Mittelachse gedreht werden, während der Konnektor (2) selbst mit eingesetzter Tulpe (19) um die Achsenmitte der Pedikelschraube (1) um 360° drehbar ist (vgl. Fig.8). Ebenso sind das Konnektorelement (9) mit eingesetzter Tulpe (19)um den kugelförmigen Pedikelschraubenkopf (18) und die Tulpe (19) um ihren Kugelkopf (21) jeweils in allen Richtungen kippbar (vgl.

Fig. 8).

In Anbetracht dessen, dass ein Verbindungsstab(3,4) vor einer Fixierung in der Tulpe(19) verschoben und gedreht werden kann (vgl. Fig. 9), ermöglicht der erfindungsgemäße Konnektor(2) somit 8 Freiheitgrade der Bewegung im nicht fixierten Zustand.

Eine weitere Ausgestaltung der Erfindung besteht darin, dass die in der ersten Aufnahme (10) befindliche Pedikelschraube (1) polyaxial beweglich, aber unlösbar mit dem Konnektorelement (9) verbunden ist, so dass die Pedikelschraube (1) gleichzeitig mit dem Konnektorelement (9) und der polyaxialen Tulpe (19), quasi als super-polyaxiale Pedikelschraube, in einen Pedikelkanal eingeschraubt werden kann.

Die folgenden Sätze fassen weitere wichtige Aspekte der Erfindung zusammen:
1. Polyaxialer Konnektor (2) für Wirbelsäulen-Fixationssysteme mit einem querverbindenden Konnektorelement (9) mit zwei Aufnahmen (10, 11) für polyaxiale Verbindungsanordnungen (7,8), wobei
   - die erste, an einem Ende des Konnektorelementes (9) befindliche und von dessen Ober- und Unterseite zugängliche Aufnahme (10) der Verbindung mit einem kugelförmigen Pedikelschraubenkopf (18) dient und im Wesentlichen als Kugelgelenklager zur Aufnahme des Pedikelschraubenkopfes (18) ausgebildet ist, der von der Unterseite des Konnektorelementes (9) einführbar ist,
      und
   - die zweite, am anderen Ende des Konnektorelementes (9) befindliche Aufnahme (11) eine zur Oberseite des Konnektorelementes (9) hinausragende, polyaxial bewegliche Tulpe (19) integriert, deren oberer Teil in bekannter Weise mit einem Feststellelement (25) zur Verbindung mit einem Verbindungsstab (3,4) eines Wirbelsäulen-Fixationssystems (5) und zur eigenen Feststellung versehen ist und deren unterer, im Konnektorelement (9) aufgenommener Teil einen Kugelkopf (21) mit einem darin beweglich gelagerten, Feder (27) beaufschlagten Druckstößel (26) aufweist,
      und
   - mit einem in dem Konnektorelement (9) zwischen den Aufnahmen (10, 11) dreh- und längsbeweglich gelagerten Klemmstück (28), über das mit Hilfe des Feststellelementes (25) und des Druckstößels (26) beim Festlegen eines Verbindungsstabes (3,4) in einem Schlitz (22) der Tulpe (19) gleichzeitig eine Klemmkraft auf den in der ersten Aufnahme (10) befindlichen Pedikelschraubenkopf (18) ausgeübt wird und über das ein Einrasten des Pedikelschraubenkopfes (18) in das Konnektorelement (9) mittels der Feder (27) ermöglicht wird.
2. Polyaxialer Konnektor (2) nach Satz 1, dadurch gekennzeichnet, dass die in der zweiten Aufnahme (11) befindliche polyaxiale Tulpe (19) ein Innengewinde und ein darin eingreifendes Feststellelement (25) mit einem Außengewinde derart aufweist, dass beim Eindrehen des Feststellelements (25) in die Tulpe (19) ein in dem Schlitz (22) der Tulpe (19) aufgenommener Verbindungsstab (3,4) festgeklemmt und der Druckstößel (26) gleichzeitig mit einer Kraft beaufschlagt wird, die in Richtung des inneren Gehäusebodens (15) des Konnektorelements (9) gerichtet ist.
3. Polyaxialer Konnektor (2) nach einem der Sätze 1 oder 2,
   dadurch gekennzeichnet, dass die zweite Aufnahme (11) des Konnektorelements (9) eine innen liegende Wulst oder eine halbringförmige Verdickung als Klemmfläche(17) aufweist.
4. Polyaxialer Konnektor (2) nach einem der Sätze 1 bis 3,
   dadurch gekennzeichnet, dass das Klemmstück (28) in Draufsicht eine im Wesentlichen H-förmige Struktur aufweist, dessen jeweils zwei beidseitige Schenkel als Laschen (29, 30) mit innen liegenden zylinderförmigen Flächen (32, 33) in Längsrichtung versehen sind, um einerseits den kugelförmigen Pedikelschraubenkopf (18) und andererseits den Kugelkopf (21) der polyaxialen Tulpe (19) aufnehmen zu können, und aus federndem Werkstoff besteht.
5. Polyaxialer Konnektor (2) nach einem der Sätze 1 bis 4,
   dadurch gekennzeichnet, dass das Gehäuse des Konnektorelements (9) im Bereich der ersten Aufnahme (10) einen im Längsquerschnitt erkennbaren, in etwa von der Mitte zur Seite hin schräg nach oben verlaufenden Abschnitt (12) des Gehäusebodens (15) aufweist.
6. Polyaxialer Konnektor (2) nach einem der Sätze 1 bis 5,
   dadurch gekennzeichnet, dass das Gehäuse des Konnektorelements (9) nach außen hin nur abgerundete Ecken aufweist.
7. Polyaxialer Konnektor( 2) nach einem der Sätze 1 bis 6,
   dadurch gekennzeichnet, dass die Verrastung , das Sichern der unverspannten Pedikelschraube (1) gegen ein Herausfallen und das Freigeben des innerhalb des Konnektorelements (9) befindlichen Klemmmechanismus mit Hilfe der am Druckstößel (26) befindlichen Feder (27), dem dreh- und längsverschiebbar gelagerten Klemmstück (28) und einer in der ersten Aufnahme (10) vorgesehenen Führungsnut (34) zum Einrasten des Pedikelschraubenkopfes (18) der Pedikelschraube (1) erzielt werden und wobei die zylinderförmigen Flächen (32) des Klemmstückes (28) für ein fühlbares Einrasten sorgen.
8. Polyaxialer Konnektor (2) nach einem der Sätze 1 bis 6,
   dadurch gekennzeichnet, dass eine in der ersten Aufnahme (10) befindliche Pedikelschraube (1) polyaxial beweglich, aber unlösbar mit dem Konnektorelement (9) verbunden ist, so dass die polyaxiale Pedikelschraube (1) gleichzeitig mit dem Konnektorelement (9) und der polyaxialen Tulpe (19) in einen Pedikelkanal eingeschraubt werden kann.
9. Wirbelsäulenfixationssystem(5) bestehend aus
   - Pedikelschrauben (1) mit Kugelkopf (21),
   - Polyaxialen Konnektoren (2) nach einem der Sätze 1 bis 7
      und
   - Verbindungsstäben (3,4).
10. Wirbelsäulenfixationssystem(5) bestehend aus
   - Polyaxialen Konnektoren (10) nach Satz 8 und
   - Verbindungsstäben (3,4).

### Bezugszeichenliste

- 1: Pedikelschraube
- 2: Konnektor
- 3, 4: Verbindungsstäbe
- 5: Wirbelsäulenfixationssystem
- 6: Pedikel
- 7, 8: Verbindungsanordnungen
- 9: Konnektorelement
- 10: 1. Aufnahme
- 11: 2. Aufnahme
- 12: Abschnitt des Gehäusebodens
- 13: U-förmige Gehäusewand
- 14: U-förmige Gehäusewand
- 15: Gehäuseboden
- 16: Klemmfläche
- 17: Klemmfläche
- 18: Pedikelschraubenkopf
- 19: Tulpe
- 20: Tulpenkopf
- 21: Kugelkopf
- 22: Schlitz
- 23: Gewinde
- 24: Bohrung
- 25: Feststellelement
- 26: Druckstößel
- 27: Feder
- 28: Klemmstück
- 29: Lasche
- 30: Lasche
- 31: Zapfen
- 32: zylinderförmige Fläche
- 33: zylinderförmige Fläche
- 34: Führungsnut

## Patentansprüche

1. Polyaxialer Konnektor (2) für Wirbelsäulen-Fixationssysteme mit einem querverbindenden Konnektorelement (9) mit zwei Aufnahmen (10, 11) für polyaxiale Verbindungsanordnungen (7,8), wobei
• die erste, an einem Ende des Konnektorelementes (9) befindliche und von dessen Ober- und Unterseite zugängliche Aufnahme (10) der Verbindung mit einem kugelförmigen Pedikelschraubenkopf (18) dient und als Kugelgelenklager zur Aufnahme des Pedikelschraubenkopfes (18) ausgebildet ist, der von der Unterseite des Konnektorelementes (9) einführbar ist,
und
• die zweite, am anderen Ende des Konnektorelementes (9) befindliche Aufnahme (11) eine zur Oberseite des Konnektorelementes (9) hinausragende, polyaxial bewegliche Tulpe (19) integriert, deren oberer Teil mit einem Feststellelement (25) zur Verbindung mit einem Verbindungsstab (3,4) eines Wirbelsäulen-Fixationssystems (5) und zur eigenen Feststellung versehen ist und deren unterer, im Konnektorelement (9) aufgenommener Teil einen Kugelkopf (21) mit einem darin beweglich gelagerten, Feder (27) beaufschlagten Druckstößel (26) aufweist,
und
• mit einem in dem Konnektorelement (9) zwischen den Aufnahmen (10, 11) dreh- und längsbeweglich gelagerten Klemmstück (28), wobei eine Klemmkraft auf den Pedikelschraubenkopf (18) mit Hilfe des Feststellelements (25) und des Druckstößels (26) mittels einer horizontalen Kraftkomponente in Richtung des Pedikelschraubenkopfes (18) aus dem Zusammenwirken des Kugelkopfes (21) und einer Klemmfläche (17) ausgeübt wird und der Pedikelschraubenkopfes (18) in dem Konnektorelement (9) mittels der horizontalen Kraftkomponente fixiert wird.

2. Polyaxialer Konnektor (2) nach Anspruch 1, **dadurch gekennzeichnet, dass** die polyaxiale Tulpe (19) ein Innengewinde und ein darin eingreifendes Feststellelement (25) mit einem Außengewinde derart aufweist, dass beim Eindrehen des Feststellelements (25) in die Tulpe (19) ein in dem Schlitz (22) der Tulpe (19) aufgenommener Verbindungsstab (3,4) festgeklemmt und der Druckstößel (26) gleichzeitig mit einer Kraft beaufschlagt wird, die in Richtung des inneren Gehäusebodens (15) des Konnektorelements (9) gerichtet ist.

3. Polyaxialer Konnektor (2) nach einem der Ansprüche 1 oder 2, **dadurch gekennzeichnet, dass** die zweite Aufnahme (11) des Konnektorelements (9) eine innen liegende Wulst oder eine halbringförmige Verdickung als Klemmfläche(17) aufweist.

4. Polyaxialer Konnektor (2) nach einem der Ansprüche 1 bis 3, **dadurch gekennzeichnet, dass** das Klemmstück (28) in Draufsicht eine im Wesentlichen H-förmige Struktur aufweist, dessen jeweils zwei beidseitige Schenkel als Laschen (29, 30) mit innen liegenden zylinderförmigen Flächen (32, 33) in Längsrichtung versehen sind, um einerseits den kugelförmigen Pedikelschraubenkopf (18) und andererseits den Kugelkopf (21) der polyaxialen Tulpe (19) aufnehmen zu können, und aus federndem Werkstoff besteht.

5. Polyaxialer Konnektor (2) nach einem der Ansprüche 1 bis 4, **dadurch gekennzeichnet, dass** der Gehäuseboden (15) des Konnektorelement (9) im Bereich der ersten Aufnahme (10) einen schräg nach oben verlaufenden Abschnitt (12) aufweist.

6. Polyaxialer Konnektor (2) nach einem der Ansprüche 1 bis 5, **dadurch gekennzeichnet, dass** das Gehäuse des Konnektorelements (9) nach außen hin nur abgerundete Ecken aufweist.

7. Polyaxialer Konnektor( 2) nach einem der Ansprüche 4 bis 6, **dadurch gekennzeichnet, dass** die Fixierung, das Sichern der unverspannten Pedikelschraube (1) gegen ein Herausfallen und das Freigeben des innerhalb des Konnektorelements (9) befindlichen Klemmmechanismus mit Hilfe der am Druckstößel (26) befindlichen Feder (27), dem dreh- und längsverschiebbar gelagerten Klemmstück (28) und einer in der ersten Aufnahme (10) vorhandenen Führungsnut (34) zum Fixieren des Pedikelschraubenkopfes (18) der Pedikelschraube (1) erzielt werden und wobei die zylinderförmigen Flächen (32) des Klemmstückes (28) für ein fühlbares Fixieren sorgen.

8. Polyaxialer Konnektor (2) nach einem der Ansprüche 1 bis 6, **dadurch gekennzeichnet, dass** eine in der ersten Aufnahme (10) befindliche Pedikelschraube (1) polyaxial beweglich, aber unlösbar mit dem Konnektorelement (9) verbunden ist, so dass die polyaxiale Pedikelschraube (1) gleichzeitig mit dem Konnektorelement (9) und der polyaxialen Tulpe (19) in einen Pedikelkanal eingeschraubt werden kann.

9. Wirbelsäulenfixationssystem(5) bestehend aus
• Pedikelschrauben (1) mit Kugelkopf (21),
• Polyaxialen Konnektoren (2) nach einem der Ansprüche 1 bis 7
und
• Verbindungsstäben (3,4).

10. Wirbelsäulenfixationssystem(5) bestehend aus
• Polyaxialen Konnektoren (10) nach Anspruch 8 und
• Verbindungsstäben (3,4).
